(19) Europäisches Patentamt
European Patent Office
Office européen des brevets

(11) Numéro de publication: **0 402 275 A1**

(12) **DEMANDE DE BREVET EUROPEEN**

(21) Numéro de dépôt: **90420239.7**

(22) Date de dépôt: **17.05.90**

(51) Int. Cl.[5]: **C08L 83/10, C08K 13/02, C08K 5/09, C08K 3/16, C08K 5/19, C02F 1/76**

(30) Priorité: **08.06.89 FR 8907831**

(43) Date de publication de la demande: **12.12.90 Bulletin 90/50**

(84) Etats contractants désignés: **AT BE CH DE DK ES FR GB GR IT LI LU NL SE**

(71) Demandeur: **RHONE-POULENC CHIMIE**
**25, quai Paul Doumer**
**F-92408 Courbevoie Cédex(FR)**

(72) Inventeur: **Prud'Homme, Christian**
**19, rue du Commandant Faurax**
**F-69006 Lyon(FR)**
Inventeur: **Torres, Ghislaine**
**104, rue Ney**
**F-69006 Lyon(FR)**

(74) Mandataire: **Seugnet, Jean Louis et al**
**RHONE-POULENC CHIMIE Service Brevets Chimie Centre de Recherches des Carrières**
**B.P. 62**
**F-69192 Saint-Fons Cédex(FR)**

(54) **Composition comprenant un copolymère silicone thermoplastique et un composé de l'iode, utilisable après sa mise en forme, pour le traitement des eaux.**

(57) La présente invention, pouvant être mise en forme par moulage ou par extrusion sous l'action de la chaleur, comprend :

A - un copolymère silicone thermoplastique,

B - au moins un composé organique et/ou minéral de l'iode sous forme solide à température ambiante, soluble dans l'eau et non toxique.

Article obtenu après mise en forme de la composition.

Utilisation de cet article pour le traitement des eaux.

EP 0 402 275 A1

# COMPOSITION COMPRENANT UN COPOLYMERE SILICONE THERMOPLASTIQUE ET UN COMPOSE DE L'IODE, UTILISABLE APRES SA MISE EN FORME, POUR LE TRAITEMENT DES EAUX

La présente invention concerne une composition à base de silicone contenant un composé de l'iode, pouvant être mise en forme, de même que l'article ainsi obtenu et son utilisation pour le traitement des eaux d'usage domestique et de boissons.

On estime actuellement à plusieurs centaines de millions le nombre de sujets présentant une défidience ou une carence en iode dans le monde. Les zones géographiques les plus touchées sont l'Amérique Latine, en particulier le long de la Cordillière des Andes, la quasi totalité des pays non côtiers de l'Afrique et l'Asie (Pakistan, Inde, Népal, Chine, Laos, etc...).

Les principales conséquences pathologiques de la déficience en iode sont bien connues. Il s'agit essentiellement d'une part du goître et ses complications parmi lesquelles on peut citer les troubles de la déglutition, les troubles respiratoires, la cancérisation, la circulation collatérale, et, d'autre part l'hypothyroïdie et ses complications parmi lesquelles on peut citer : le crétinisme, les désordres cérébraux, les accouchements prématurés, les fausses couches et les anomalies congénitales.

Si la carence en iode a disparu des pays industrialisés grâce à l'iodation du sel de cuisine, il n'en est pas de même dans les pays en voie de développement où les principales actions menées jusqu'alors sont demeurées inefficaces.

Ces actions visent essentiellement d'une part :

- l'iodation du sel de cuisine : elle est inopérante dans la plupart des pays en voie de développement car bien souvent la consommation de sel est minime, les circuits de distribution du sel à travers des réseaux économiques et commerciaux sont quasiment inexistants et, enfin, en région tropicale, l'iode rajouté au sel s'en échappe rapidement lorsqu'il n'est pas parfaitement emballé.

et d'autre part :

- l'injection intramusculaire d'huile iodée : cette injection a l'avantage de présenter une action différée (action retard) mais elle n'est pas sans présenter des inconvénients, en particulier les risques d'infection, les risques d'allergie de l'iode, les risques d'hyperthyroïdie ou d'hypothyroïdie induites par l'injection d'une dose nécessairement supra-physiologique.

La présente invention a pour but de proposer une composition à base de silicone contenant de l'iode, qui après mise à la forme voulue est utilisable pour le traitement continu des eaux d'usage domestique, en particulier dans les systèmes d'adduction et de traitement des eaux dans les puits et les forages et qui permette de libérer une quantité contrôlée et adaptée d'iode en vue d'assurer d'une part le traitement collectif des diverses manifestations dues à une carence en iode et d'autre part une prophylaxie de ces diverses manifestiations.

Elle a également pour but de proposer une composition de silicone contenant de l'iode qui après avoir été mise à la forme voulue et immergée de façon appropriée dans les endroits contenant l'eau à traiter, en particulier les puits et forages, libère de façon continue, de préférence pendant au moins un an, une quantité appropriée d'iode sous une forme et à une dose thérapeutiquement active et efficace pour soigner les diverses maladies dues à une carence en iode.

Elle a également pour but l'obtention d'articles mis à la forme appropriée qui peuvent être introduits et chargés aisément dans les puits et/ou forages où se trouve l'eau à traiter.

Il est connu par les demandes de brevets français publiées n° 2 611 733 et 2 611 734 de la Société Rhône-Poulenc des compositions contenant, en plus du composé de l'iode, au moins un polyorganosiloxane sous forme d'huile ou de gomme, ainsi qu'un catalyseur pour l'obtention d'élastomères à partir de ces compositions. Ces compositions permettent d'atteindre les buts ci-avant. Toutefois les articles élastomériques contenant un composé de l'iode obtenus à partir de ces compositions brevetées présentant l'inconvénient de renfermer les catalyseurs nécessaires à leur obtention.

Un but de la présente invention est donc l'obtention de compositions contenant un composé de l'iode dans lesquelles le produit polymére utilisé ne comprend pas d'impuretés indésirables, telles que par exemple des catalyseurs.

Un autre but de la présente invention est l'obtention de composition ne comprenant pas, en plus du composé de l'iode, que des produits polymères à base de silicones.

Un autre but de la présente invention est l'obtention de compositions dans lesquelles le produit polymére peut être purifié avant sa mise en forme, par exemple par solubilisation dans des solvants organiques puis reprécipitation (dans un non solvant), avant d'être mélangé au composé de l'iode.

Un autre but de la présente invention est une composition dans laquelle le polymère utilisé avec le composé de l'iode est soluble dans certains solvants organiques.

Un autre but de la présente invention est une composition dans laquelle le composé de l'iode peut être réparti de façon homogène, par agitation, dans une solution du polymère utilisé.

Un autre but de la présente invention est l'obtention de compositions permettant la préparation aisée d'articles en forme, par moulage ou par extrusion, sous l'action de la température.

Un autre but de la présente invention est la préparation d'articles en forme obtenus à partir de telles compositions, ces articles, une fois mis dans l'eau, libérant le composé de l'iode suivant une cinétique d'ordre O et de façon continue jusqu'à ce que 80 % en poids et plus du composé iodé soit libéré.

Ces buts et d'autres sont atteints par la présente invention qui concerne une composition pouvant être mis en forme par moulage ou par extrusion sous l'action de la chaleur, caractérisé en ce qu'elle comprend :

A - un copolymère silicone thermoplastique,

B - au moins un composé organique et/ou minéral de l'iode sous forme solide à température ambiante, soluble dans l'eau et non toxique.

Un autre objet de la présente invention concerne l'article mis en forme obtenu avec une composition définie ci-avant, ainsi que le procédé de traitement des eaux dans lequel on utilise l'article mis en forme.

Les copolymères silicones thermoplastiques utilisables dans le cadre de la présente invention sont choisis parmi les copolymères à blocs :

- soit multi-séquencés linéaires, dont la chaîne polymère principale sensiblement linéaire est constituée par une alternance de segments ou blocs polydiorganosiloxanes et de segments ou blocs organiques,
- soit greffés, constitués par une chaîne polydiorganosiloxane sur laquelle sont greffés des chaînes organiques.

Les radicaux organiques des motifs diorganosiloxyle sont de préférence des radicaux alkyle en $C_1$-$C_4$, en particulier méthyle, des radicaux trifluoro-3,3,3-propyle et des radicaux phényle.

Les copolymères thermoplastiques utilisables dans le cadre de la présente invention sont en outre des polymères qui se ramollissent sous l'effet de la chaleur, qui sont solubles dans certains solvants organiques et dont la mise en forme met en jeu des processus physiques réversibles.

Les copolymères thermoplastiques préférés sont ceux qui présentent une Tg (température de transition vitreuse) ou température de fusion (pour les copolymères semi-cristallins) supérieures à la température ambiante (25° C) et, de préférence supérieures à 40° C et généralement inférieures à 200° C.

Les copolymères silicones thermoplastiques utilisables dans le cadre de la présente invention sont avantageusement solubles dans au moins un des solvants organiques ou un mélange de solvants organiques choisis parmi le chloroforme, l'acétone, la méthyléthylcétone, le tétrahydrofurane, le dichloroéthane, le tétrachloroéthane, le tétrachlorure de carbone, le trichloroéthylène, l'hexane, l'heptane, le méthanol, l'éthanol, l'isopropanol et le toluène.

Les segments séquencés, blocs ou greffons organiques des copolymères silicones thermoplastiques peuvent être en particulier :

- des polyuréthannes (voir par exemple le brevet canadien CA-A1 072 241, les brevets américains US-A-4 145 508, US-A-4 180 515 et US-A-4 516 758),
- des polysilarylènes (voir par exemple US-A-4 233 427, FR-A-2 407 950 et FR 1 299 160),
- des polystyrènes (voir par exemple US-A-4 263 401),
- des polyesters (voir par exemple US-A-3 701 815),
- des polyéthers,
- des polycarbonates (voir par exemple J. Polym. Sci., Polymer Letters ed. 7, p. 569-577) (1969),
- des polyamides,
- des polyimides,
- des polyimides/amides,
- des polysulfones,
- des polyacrylates,
- des polyméthacrylates,

et de façon générale les copolymères silicones thermoplastiques décrits aux pages 181 à 198 de l'édition 1988 de INORGANIC and ORGANOMETALLIC POLYMERS.

Ainsi les copolymères silicones thermoplastiques selon la présente invention peuvent notamment être ceux décrits dans les brevets cités, ci-avant, incorporés comme références dans la présente demande.

A titre illustratif, ces copolymères peuvent être notamment (selon le brevet US-A-4 233 427 cité ci-avant) ceux ayant une pluralité de motifs récurrents répondant à l'une et/ou à l'autre des deux formules F1 et F'1 :

F1 : $\{ A[SiR_2(GSiR_2)_aASiR_2G'SiR_2A]_p\ SiR_2(GSiR_2)_aASiR_2(OSiR_2)_n \}$

F'1 : $\{ A[SiR_2G'SiR_2ASiR_2(GSiR_2)_aA]_p\ SiR_2G'SiR_2ASiR_2(OSiR_2)_n \}$

dans lesquelles les différents symboles ont la signification suivante :

- les symboles A, identiques, représentent des radicaux alcoylènes, linéaires ou ramifiés, ayant de 2 à 6 atomes de carbone, des radicaux cyclohexylènes ;
- les symboles R, identiques ou différents, représentent :
. des radicaux alcoyles et halogénoalcoyles ayant de 1 à 5 atomes de carbone,
. des radicaux cycloalcoyles et halogénocycloalcoyles ayant de 3 à 8 atomes de carbone,
. des radicaux aryles et halogénoaryles ayant de 6 à 8 atomes de carbone,
. des radicaux cyanoalcoyles ayant de 3 à 4 atomes de carbone ; - les symboles G, identiques, représentent :
. des radicaux alcoylènes, linéaires ou ramifiés, ayant de 1 à 6 atomes de carbone,
. des radicaux organiques divalents répondant à la formule $F_2$ : $-(CH_2)_xQTQ(CH_2)_x-$ dans laquelle :
+ les symboles Q, identiques, représentent l'un des groupes -O-, -OCO-(-OCO-étant lié à T par le radical -CO-),
+ le symbole T représente un radical hydrocarboné divalent, monocyclique, ayant de 6 à 8 atomes de carbone, ou un radical organique divalent ayant de 10 à 22 atomes de carbone constitué de 2 cycles hydrocarbonés soudés l'un à l'autre ou liés par une liaison valentielle ou par l'un des groupes de formules -O- ; $-CH_2-$ ; $-C(CH_3)_2-$ ; $-Si(R')_{-2}$, R′ étant un radical alcoyle ayant de 1 à 3 atomes de carbone ;
+ les symboles x, identiques, représentent 1, 2 ou 3, ou
. des radicaux hydrocarbonés divalents répondant à la formule $F_3$: $(CH_2)_bT(CH_2)_b$ dans laquelle le symbole T a la signification donnée dans la formule $F_2$, et les symboles b, identiques, représentent 0 ou 1 ;
- les symboles G′, identiques, ont la signification donnée pour G sauf qu'ils ne répondent pas à la formule $F_2$ ;
- les symboles a, identiques, représentent 0 ou 1 ;
- le symbole p représente un nombre quelconque allant de 1 à 120 ;
- le symbole n représente un nombre quelconque allant de 1 à 1500.

Dans les compositions selon la présente invention, les segments, blocs ou greffons organiques sont présents suivant une teneur pondérale de 5 à 60 %, de préférence de 10 à 40 % par rapport au poids total du copolymère silicone thermoplastique.

Comme composé minéral de l'iode, à l'exception de l'iode moléculaire $I_2$, on peut utiliser seul ou en mélange :
- les iodures ou iodates de formules générales :
$(M^{a+})(I^-)_a$
et $(M^{a+})(IO_3)_a$
dans lesquelles a est un nombre entier supérieur ou égal à 1 et M est un cation qui peut être choisi parmi un métal alcalin tel que le sodium et le potassium, un métal alcalino-terreux tel que le magnésium et le calcium, un métal de transition tel que le fer et le manganèse, un ammonium quaternaire $(NY_4)^+$, dont les radicaux Y identiques ou différents représentent un radical alkyle linéaire ou ramifié en $C_1$-$C_{20}$ ou un atome d'hydrogène, tel que l'ion ammonium $NH^+_4$.

Les cations $M^{a+}$ et $NY_4^+$, sont choisis de telle sorte que l'iodure ou l'iodate correspondant soit un solide ou un liquide à température ambiante, soit soluble dans l'eau et soit non toxique.

Comme iodures et iodates on peut en particulier utiliser ceux de formules :
Na I , Na $IO_3$,
K I , K $IO_3$,
Mg $I_2$ , Mg $I_2$, $8H_2O$,
$Mg(IO_3)_2$, $4H_2O$,
$NH_4I$
Fe $I_2$, $4H_2O$
Mn $I_2$

Ces sels peuvent contenir de l'eau d'hydratation ou de l'eau de constitution.

Comme composé de l'iode à la fois organique et minéral on peut par exemple utiliser l'iodobéhénate de calcium de formule :
$(C_{21}H_{42}ICO_2)_2Ca$

Comme composé organique de l'iode on peut citer la polyvinylpyrrolidone iodée.

Pour des raisons de facilité de mise en oeuvre les composés d'iode solides sont préférés, et parmi ceux-ci NaI et $KIO_3$.

Tous les composés de l'iode tels que définis au-dessus, lorsqu'ils sont en solution dans l'eau à traiter, libèrent l'iode sous une forme non toxique et thérapeutiquement efficace. Par composé de l'iode non toxique on entend selon l'invention un composé qui, en solution, n'est pas toxique aux posologies préconisées par la présente invention.

Par composé de l'iode soluble dans l'eau on entend un composé présentant une solubilité d'au moins 500 mg/l à température ambiante.

On utilise, dans la composition selon la présente invention, généralement de 5 à 130 parties (en poids), de préférence de 10 à 90 parties de composé de l'iode (B) pour 100 parties du copolymère silicone thermoplastique (A).

En particulier dans les pays en développement, l'eau à usage domestique (boisson, lavage, irrigation, etc...) est pour l'essentiel apportée par deux types de structure, les puits et les forages.

Pour des raisons évidentes de coût, d'efficacité, de salubrité, la création nouvelle d'un point d'eau se fait souvent par forage.

Un forage est une colonne d'air forée à tavers des roches compactes ayant une profondeur comprise généralement entre 20 et 100 mètres et un diamètre d'au moins environ 10 cm. L'eau s'infiltre dans cette colonne, par les fissures ou les divers interstices. La réserve d'eau immédiatement disponible est donc constituée d'une colonne de 10 à 70 mètres, généralement de 30 à 50 mètres de haut qui est extraite par exemple à l'aide d'une pompe à corps immergé.

Cette eau se renouvelle principalement en fonction de l'utilisation du forage qui dépend de la saison. En effet en saison des pluies le forage est traditionnellement moins utilisé. Par contre en saison sèche, le forage débite environ 10-12 heures par jour, soit une quantité comprise entre 5 et 10 $m^3$ par jour pendant environ six mois.

En règle générale, un puits peut être asséché deux fois durant la journée au moment de la saison sèche ce qui correspond avec ces données statistiques moyennes, à un maximum d'utilisation de 5 à 10 $m^3$.

De nombreuses études montrent que dans les zones de grande endémie goîtreuse, le taux pré-existant d'équivalent en iode dans l'eau des forages ou des puits est inférieur à 2 microgrammes par litre (2 $\mu$g/l). Il est estimé actuellement qu'un apport journalier d'environ 100 $\mu$g d'équivalent d'iode par jour et par personne serait suffisant pour prévenir le développement d'un goître endémique et sans doute environ 150 $\mu$g lorsqu'il y a consommation régulière de substances goitrigènes. A l'inverse une intoxication aigüe à l'iode peut être responsable d'irritation neurologique, d'hyperthyroïdie ou d'hypothyroïdie.

Il est admis en pratique médicale que l'absorption d'une dose de 3 grammes d'équivalent d'iode par un sujet adulte en une seule prise n'entraîne pas d'effet secondaire.

Par conséquent, pouvoir apporter à un individu de 20 à 200 $\mu$g de préférence environ 100 $\mu$g, d'équivalent iode par jour constitue le but recherché.

Ainsi sachant qu'un individu adulte absorbe en moyenne 2 litres d'eau par jour et sur la base des données ci-dessus (forage ayant un débit de 600 l/h), il apparaît souhaitable qu'un litre d'eau traitée contienne environ 50 $\mu$g/l d'iode ce qui correspond à 50 $\mu$g, d'équivalent iode par litre et par personne, ce qui nécessite que l'élastomère de silicone libère 720 mg/j d'équivalent d'iode, soit 270 g d'équivalent iode à libérer pendant une année.

Le système de libération contrôlée de l'iode fait partie des systèmes matriciels dont la diffusion du principe actif est normalement régie par la loi de FICK, c'est-à-dire par une cinétique de diffusion d'ordre 1/2 pour seulement 60 % en poids du principe actif. Au delà de 60 % il y a épuisement de la matrice et les flux de diffusion sont fortement diminués. De façon surprenante et inattendue, on a trouvé que le système matriciel avec un copolymère silicone thermoplastique conforme à l'invention libère l'iode suivant une cinétique d'ordre 0 et de façon continue et cela jusqu'à ce que 80 % en poids et plus du composé d'iode soit libéré.

L'avantage considérable apporté par la matrice en copolymère silicone thermoplastique et donc qu'il est très facile d'extrapoler la diffusion continue du principe actif après une mesure de la quantité libérée au bout d'au moins un mois puisque l'on sait que la cinétique de diffusion est d'ordre 0 et qu'au moins 80 % du composé d'iode sera libéré suivant cette cinétique.

De façon à maîtriser la libération du principe actif, il est avantageux de présenter la matrice en copolymère silicone thermoplastique sous la forme de modules ou articles élémentaires de formes variées tel que des cubes, des parallélépipèdes rectangles, des cylindres, des sphères dont les paramètres fondamentaux sont les suivants :

- la nature du composé d'iode,
- le diamètre moyen (granulométrie) g des particules du composé d'iode dans le cas préféré où ce dernier est un solide,
- la teneur t de composé d'iode au sein de la matrice,
- le rapport R de la surface au volume du module.

La nature du composé d'iode et sa granulométrie définissent la vitesse de diffusion v à travers la matrice, du principe actif.

Plus g est petit et plus v est lent et inversement.

Plus t est grand et plus le flux de principe actif est élevé et inversement.

Plus R est grand et plus le flux de principe actif est grand et inversement.

L'homme de métier, par des expériences de routine, peut sans difficulté aboutir rapidement au résultat visé en extrapolant le temps d'élution théorique qui correspondra au temps réel de diffusion du principe actif.

Pour NaI et $KIO_3$ qui sont les composés d'iode préférés, g, t et R peuvent être avantageusement choisis dans les zones suivantes :

- g compris entre 1 et 500 $\mu$m,
- t compris entre 10 et 100 parties en poids de composé d'iode pour 100 parties de (A),
- R compris entre 0,5 et 50 pour une forme cylindrique.

Il est en outre souhaitable que le composé d'iode soit dispersé de façon homogène au sein de la matrice.

Pour celà, dans un (ou un mélange de) solvant(s) organique(s) approprié(s) choisi(s) parmi ceux cités plus avant, on solubilise le copolymère silicone thermoplastique, éventuellement en chauffant, puis lorsque la dissolution est complète on ajoute sous agitation le composé de l'iode que l'on répartit de façon homogène dans la solution. On évapore alors le solvant organique et la composition obtenue est alors mise en forme par chauffage et l'on obtient, par exemple par moulage ou par extrusion, des articles contenant la composition selon la présente invention.

Eventuellement ces articles peuvent être obtenus par chauffage, par exemple par moulage ou par extrusion, après que la composition ait été préparée directement par malaxage du copolymère silicone thermoplastique finement divisé avec le composé solide de l'iode sous forme de poudre, jusqu'à l'obtention d'un mélange homogène. Il n'est alors pas nécessaire d'effectuer une solubilisation du copolymère silicone thermoplastique.

EXEMPLE 1 :

1.1 - Préparation du copolymère silicone thermoplastique :

On utilise un copolymère polyséquencé thermoplastique dont la préparation est décrite à l'exemple 4 du brevet USA 4 233 427, l' $\alpha$-$\omega$ dihydrogénopolydiméthyl siloxane utilisé ayant une masse moléculaire moyenne en nombre de 22 800 g/mole, déterminée par dosage des fonctions SiH.

Ce copolymère est formé par une alternance de segments polydiméthylsiloxane et de segments poly [-(diméthylsilylène)phénylène-(diméthylsilylène)-1,2-éthanediyl)]répondant à la formule générale moyenne :

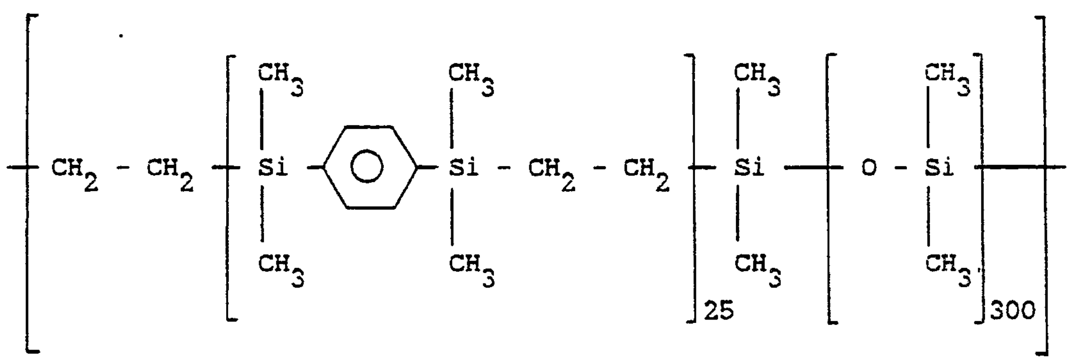

La proportion de segments polydiméthylsiloxane dans ce copolymère est de 80 % en poids.

La viscosité inhérente de ce copolymère est de 0,44 dl/g (mesurée à 25 °C sur une solution dans le chloroforme ayant une concentration de 3 g/dl.

1.2 - Préparation d'une composition selon la présente invention avec le copolymère préparé ci-avant :

On dissout 100 g du copolymère dans 70 g de toluène anhydre à une température proche de 110 °C.

Quand le mélange est homogène on ajoute 25 g de NaI, de granulométrie inférieure à 50 µm (microns). Le mélange est agité pendant 30 mn de manière à disperser le NaI (non soluble dans le toluène) de façon homogène dans le copolymère.

Le toluène est ensuite éliminé à l'étuve pendant 5 heures.

1.3 - Préparation d'un article, par mise en forme de la composition ci-avant :

La composition préparée en 1.2 est alors introduite dans un tube de diamètre égal à 2,3 cm et mise à l'étuve. La température est ensuite montée à 210 °C (température de ramollissement du copolymère) et maintenue pendant 2 heures.

1.4 - Protocole expérimental de la mesure de la cinétique d'élution

L'article précédemment préparé est découpé à la longueur désirée (50 mm), conformément au rapport Surface/Volume (2,14 cm-1) que l'on désire atteindre, et immergé dans un récipient contenant 600 ml d'eau distillée, thermostatée à 20 °C.

Le récipient est équipé d'un système d'agitation magnétique, animé d'un mouvement de rotation lent (100 tr/min) assurant l'homogénéité de la solution. Il est recouvert d'un couvercle afin de minimiser l'évaporation de l'eau.

Des prélèvements journaliers de 1 ml sont réalisés dans les premiers temps de l'élution, et hebdomadaires au bout de 15 jours d'élution.

La concentration en iodure ou iodate, libérée par jour, est déterminée par un dosage avec une électrode spécifique à iodure.

A un millilitre de prélèvement du récipient on rajoute deux millilitres d'une solution (K2SO4 + acide ascorbique) - cette solution joue le rôle de tampon ionique, et de solution réductrice dans le cas du dosage des iodates - et un millilitre d'eau distillée. On immerge l'électrode dans cette solution et on lit le potentiel électrochimique de la solution. Une courbe d'étalonnage préalablement établie avec des solutions en iodure de 5.10-5 M/l (M : mole) à 5.10-2 M/l permet de calculer la concentration (C) en iodure ou iodate en mg/l de la solution.

Les caractéristiques du cylindre immergé (de l'article utilisé pour faire cette mesure) sont :

. Diamètre = 23 mm
. Hauteur = 50 mm
. Surface = 44,4 cm2
. Volume = 20,76 cm3
. S/V = 2,14 cm-1
. Masse totale = 23,17 g
. Quantité initiale de I (Qo) = 3,92 g.

Dans le tableau suivant (Tableau 1) sont rassemblés les résultats de la cinétique d'élution.

Q cumulé correspond à la quantité d'équivalent $I^-$ (que nous appelons ion actif) éluée à l'instant t.

Sachant que 80 % en moles de l'ion actif incorporé élue selon une cinétique d'ordre zéro en fonction du temps, nous pouvons calculer pour chaque exemple le temps d'élution théorique (Te) selon la formule :

$$Te = \frac{0,8 \times Qo}{\text{Flux journalier}}$$

(Te étant exprimé en jours)

TABLEAU 1

| Temps (jour) | Q cumulé (mg $I^-$) | 100 x Q/Qo (%) |
|---|---|---|
| 0,4 | 72 | 1,79 |
| 1 | 77 | 1,92 |
| 2 | 66 | 1,70 |
| 3 | 144 | 3,6 |
| 6 | 187 | 4,7 |
| 7 | 243 | 6,25 |
| 9 | 254 | 6,53 |
| 16 | 315 | 7,97 |
| 23 | 453 | 11,52 |
| Te = 150 jours | | |

EXEMPLE 2 :

2.1 - On utilise le copolymère préparé ci-avant en 1-1.

2.2 - On prépare une composition de la même manière et avec les mêmes quantités de produits qu'en 1.2, si ce n'est que l'iodure de sodium (NaI) utilisé a une granulométrie comprise entre 100 et 200 µm (microns).

2.3 - On mette cette composition en forme (par moulage), pour obtenir un article, de la même manière qu'en 1.3.

2.4 - On mesure (selon 1.4) la cinétique d'élution en produit iodé de l'article préparé ci-avant et découpé à la longueur voulue (50 mm) conformément au rapport surface/volume (2,14 $cm^{-1}$) que l'on désire atteindre.

Les caractéristiques du cylindre immergé sont :

. Diamètre = 23 mm
. Hauteur = 50 mm
. Surface = 44,4 cm2
. Volume = 20,76 cm3
. S/V = 2,14 cm-1
. Mass totale = 22,5 g
. Quantité initiale de I (Qo) = 3,81 g.

Dans le tableau 2 ci-après sont rassemblés les résultats de la cinétique d'élution.

TABLEAU 2

| Temps (jour) | Q cumulé (mg $I^-$) | 100 x Q/Qo (%) |
|---|---|---|
| 0,4 | 149 | 3,88 |
| 1 | 259,5 | 6,76 |
| 2 | 381 | 9,99 |
| 3 | 375 | 9,90 |
| 6 | 513,5 | 13,51 |
| 7 | 530 | 13,92 |
| 9 | 579,8 | 15,18 |
| 16 | 728,8 | 19,16 |
| 23 | 1049 | 27,48 |
| Te = 80 jours | | |

EXEMPLE 3 :

3.1 - On utilise le copolymère préparé en 1.1.

3.2 - On prépare une composition de la même façon et avec les mêmes quantités de produits qu'en 1.2, si ce n'est que l'iodure de sodium a une granulométrie comprise entre 200 et 400 µm (microns).

3.3 - On prépare un article, par mise en forme de la composition par moulage, comme en 1.3.

3.4 - On mesure (selon protocole décrit en 1.4) la cinétique d'élution de l'article (cylindre) préparé en 3.3 et découpé à la longueur désirée (50 mm), conformément au rapport surface/volume (2,14 $cm^{-1}$) que l'on désire atteindre.

Les caractéristiques du cylindre immergé sont :

. Diamètre = 23 mm
. Hauteur = 50 mm
. Surface = 44,4 cm2
. Volume = 20,76 cm3
. S/V = 2,14 cm-1
. Masse totale = 23,04 g
. Quantité initiale de I (Qo) = 3,9 g.

Dans le tableau 3 ci-après sont rassemblés les résultats de la cinétique d'élution.

TABLEAU 3

| Temps (jour) | Q cumulé (mg $I^-$) | 100 x Q/Qo (%) |
|---|---|---|
| 0,4 | 132,5 | 3,4 |
| 1 | 149 | 3,79 |
| 2 | 303,7 | 7,83 |
| 3 | 386,5 | 9,84 |
| 6 | 519 | 13,36 |
| 7 | 519 | 13,25 |
| 9 | 646 | 16,51 |
| 16 | 833,7 | 21,38 |
| 23 | 1225,8 | 31,46 |
| Te = 58 jours | | |

EXEMPLE 4 :

4.1 - On utilise le copolymère préparé en 1.1

4.2 - On prépare une composition de la même manière qu'en 1.2, mais toutes les quantités de produits utilisés sont divisés par deux, l'iodure de sodium étant remplacé par de l'iodate de potassium de granulométrie inférieure à 50 µm (microns).

4.3 - On prépare un article de la même manière qu'en 1.3, mais le tube utilisé a un diamètre de 10 mm.

4.4 - On mesure (selon le protocole décrit en 1.4) la cinétique d'élution en produit iodé de l'article (cylindre) préparé en 4.3 et découpé à la longueur désirée (20 mm) conformément au rapport surface/volume (4,7 $cm^{-1}$) que l'on désire atteindre.

Les caractéristiques du cylindre immergé sont :

. Diamètre = 10,8 mm
. Hauteur = 22,5 mm
. Surface = 8,6 cm2
. Volume = 1,83 cm3
. S/V = 4,7 cm-1
. Masse totale = 2,062 g
. Quantité initiale de I (Qo) = 0,245 g.

Dans le tableau 4 ci-après sont rassenblés les résultats de la cinétique d'élution.

TABLEAU 4

| Temps (jour) | Q cumulé (mg $I^-$) | 100 x Q/Qo (%) |
|---|---|---|
| 1 | 2,91 | 1,19 |
| 2 | 3,85 | 1,57 |
| 3 | 3,82 | 1,56 |
| 6 | 4,72 | 1,93 |
| 7 | 5,63 | 2,30 |
| 9 | 6,52 | 2,66 |
| 16 | 6,44 | 2,63 |
| 23 | 9,14 | 3,73 |
| Te = 768 jours | | |

**Revendications**

1. - Composition pouvant être mise en forme par moulage ou par extrusion sous l'action de la chaleur, caractérisée en ce qu'elle comprend:

A - un copolymère silicone thermoplastique,

B - au moins un composé organique et/ou minéral de l'iode sous forme solide à température ambiante, soluble dans l'eau et non toxique.

2. - Composition selon la revendication 1, caractérisée en ce que le copolymère silicone thermoplastique est choisi parmi les copolymères à blocs :
- soit multi-séquencés linéaires, dont la chaîne polymère principale sensiblement linéaire est constituée par une alternance de segments ou blocs polydiorganosiloxanes et de segments ou blocs organiques,
- soit greffés, constitués par une chaîne polydiorganosiloxane sur laquelle sont greffés des chaînes organiques.

3. - Composition selon la revendication 1 ou 2, caractérisée en ce que les séquences, blocs ou greffons du copolymère silicone thermoplastique sont choisis parmi les polyuréthannes, les polysilarylènes, les polystyrènes, les polyesters, les polyéthers, les polycarbonates, les polyamides, les polyimides, les polyimides/amides, les polysulfones, les polyacrylates et les polyméthacrylates.

4. - Composition selon la revendication 3, caractérisée en ce que la teneur pondérale des séquences, blocs ou greffons organiques est comprise entre 5 et 60 %.

5. - Composition selon l'une quelconque des revendications précédentes, caractérisée en ce que le copolymère silicone thermoplastique présente une température de transition vitreuse ou une température de fusion supérieure à 40 °C.

6. - Composition selon l'une quelconque des revendications précédentes, caractérisée en ce que le copolymère silicone thermoplastique est constitué d'une pluralité de motifs récurrents répondant à l'une et/ou à l'autre des deux formules F1 et F'1 :

F1 : $\{ A[SiR_2(GSiR_2)_aASiR_2G'SiR_2A]_pSiR_2(GSiR_2)_aASiR_2(OSiR_2)_n \}$

F'1 : $\{ A[SiR_2G'SiR_2ASiR_2(GSiR_2)_aA]_pSiR_2G'SiR_2ASiR_2(OSiR_2)_n \}$

dans lesquelles les différents symboles ont la signification suivante :
- les symboles A, identiques, représentent des radicaux alcoylènes, linéaires ou ramifiés, ayant de 2 à 6 atomes de carbone, des radicaux cyclohexylènes ;
- les symboles R, identiques ou différents, représentent :
. des radicaux alcoyles et halogénoalcoyles ayant de 1 à 5 atomes de carbone,
. des radicaux cycloalcoyles et halogénocycloalcoyles ayant de 3 à 8 atomes de carbone,
. des radicaux aryles et halogénoaryles ayant de 6 à 8 atomes de carbone,
. des radicaux cyanoalcoyles ayant de 3 à 4 atomes de carbone ; - les symboles G, identiques, représentent :

. des radicaux alcoylènes, linéaires ou ramifiés, ayant de 1 à 8 atmes de carbone,

. des radicaux organiques divalents répondant à la formule $F_2$ : $-CH_2)_xQTQ(CH_2)_x-$ dans laquelle :

+ les symboles Q, identiques, représentent l'un des groupes -O-, -OCO-(-OCO-étant lié à T par le radical -CO-),

+ le symbole T représente un radical hydrocarboné divalent, monocyclique, ayant de 6 à 8 atomes de carbone, ou un radical organique divalent ayant de 10 à 22 atomes de carbone constitué de 2 cycles hydrocarbonés soudés l'un à l'autre ou liés par une liaison valentielle ou par l'un des groupes de formules -O- ; $-CH_2-$ ; $-C(CH_3)_2-$ ; $-Si(R')-_2$, R' étant un radical alcoyle ayant de 1 à 3 atomes de carbone ;

+ les symboles x, identiques, représentent 1, 2 ou 3, ou

. des radicaux hydrocarbonés divalents répondant à la formule $F_3$: $(CH_2)_bT(CH_2)_b$ dans laquelle le symbole T a la signification donnée dans la formule $F_2$, et les symboles b, identiques, représentent 0 ou 1 ;

- les symboles G', identiques, ont la signification donnée pour G sauf qu'ils ne répondent pas à la formule $F_2$ ;

- les symboles a, identiques, représentent 0 ou 1 ;

- le symbole p représente un nombre quelconque allant de 1 à 120 ;

- le symbole n représente un nombre quelconque allant de 1 à 1500.

7. - Composition selon la revendication 6 caractérisée en ce que le copolymère silicone thermoplastique est constitué d'une pluralité de motifs récurrents répondant à la formule

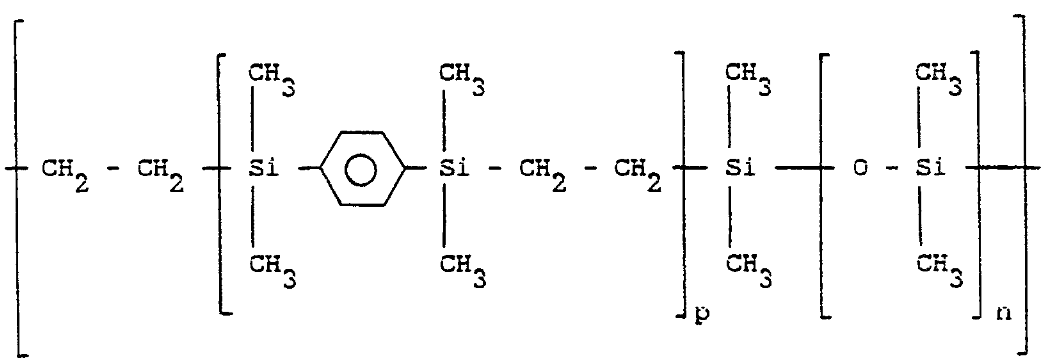

8. - Composition selon l'une quelconque des revendications précédentes, caractérisée en ce qu'elle contient, en poids, de 5 à 130, de préférence de 10 à 90 parties de composé de l'iode (B) pour 100 parties du copolymère silicone thermoplastique (A).

9. - Composition selon l'une quelconque des revendications précédentes, caractérisée en ce que le composé de l'iode (B) est un iodure ou iodate de formules générales :

$M^{a+}) (I^-)a$

et $(M^{a+}) (IO^-{}_3)a$

dans lesquelles a est un nombre entier supérieur ou égal à 1 et M est un cation choisi parmi un métal alcalin, un métal alcalino-terreux, un métal de transition et un ammonium quaternaire $(NY_4)^+$ dont les radicaux Y identiques ou différents représentent un radical alkyle linéaire ou ramifié en $C_1$-$C_{20}$ ou un atome d'hydrogène.

10. - Composition selon l'une quelconque des revendications précédentes, caractérisée en ce que le composé de l'iode (B) est choisi parmi

Na I , Na $IO_3$,

K I , K $IO_3$,

Mg $I_2$ , Mg $I_2$, $8H_2O$,

$Mg(IO_3)_2$, $4H_2O$,

$NH_4I$

Fe $I_2$, $4H_2O$

Mn $I_2$

11. - Composition selon l'une quelconque des revendications 1 à 8, caractérisée en ce que le composé de l'iode (D) est d'iodobéhénate de calcium.

12. - Composition selon l'une quelconque des revendications 1 à 8, caractérisée en ce que le composé de l'iode (D) est la polyvinylpyrrolidone iodée.

13. - Article mis en forme obtenu avec une composition selon l'une quelconque des revendications précédentes.

14. - Procédé de traitement des eaux, caractérisé en ce que l'on immerge une quantité adaptée de l'article tel que défini à la revendication 13, en vue de libérer dans l'eau une quantité continue et contrôlée d'iode.

Office européen des brevets

# RAPPORT DE RECHERCHE EUROPEENNE

Numero de la demande

EP 90 42 0239

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5) |
|---|---|---|---|
| A | EP-A-0 284 521 (RHONE-POULENC) * page 2, ligne 1 - page 5, ligne 22; exemples; revendications 1,3-6,12 * | 1,9-14 | C 08 L 83/10<br>C 08 K 13/02<br>C 08 K 5/09<br>C 08 K 3/16<br>C 08 K 5/19<br>C 02 F 1/76 |
| A | EP-A-0 283 407 (RHONE-POULENC) * page 2, ligne 1 - page 5, ligne 6; exemples; revendications 1,3-6,14 *; FR-A-2611733 (cat. D) | 1,9-14 | |
| A | EP-A-0 283 408 (RHONE-POULENC) * pages 1-4; exemples; revendications 1-5,14 *; & FR-A-2611734 (cat. D) | 1,9-14 | |
| A | DE-A-2 707 549 (AQUANORT INGENIEUR SKIRDE & CO. et al.) * revendications 1,2,5,11 * | 1,14 | |
| A | US-A-3 907 720 (N.D. FIELD et al.) * abrégé; revendications 1,9 * | 1,14 | |
| | | | **DOMAINES TECHNIQUES RECHERCHES (Int. Cl.5)** |
| | | | A 61 K<br>C 02 F<br>C 08 K |

Le présent rapport a été établi pour toutes les revendications

| Lieu de la recherche | Date d'achèvement de la recherche | Examinateur |
|---|---|---|
| BERLIN | 23-08-1990 | HOEPFNER W.W.G. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P0402)